# EUROPEAN PATENT APPLICATION

(11) **EP 2 689 792 A1**
(43) Date of publication of application: **29.01.2014**
(21) Application number: 13178132.0
(22) Date of filing: 26.07.2013
(51) Int. Cl.: A61M 1/16

(54) **extracorporeal circuit for removing CO2 from blood**

(30) Priority: 26.07.2012 IT BO20120405
(71) Applicant: Patroniti, Nicolo Antonino, Lissone (IT); Pesenti, Antonio, Milano (IT)
(72) Inventor: Patroniti, Nicolo Antonino, Lissone (IT); Pesenti, Antonio, Milano (IT)
(74) Representative: Bosman, Cesare

(57) **Abstract**

An extracorporeal circuit (1) for removing CO₂ from blood comprising a blood taking line (2) for taking blood from the patient, an oxygenation assembly (4) and a blood reintroduction line (5) for re-introducing blood into the patient. The oxygenation assembly (4) comprises a first dialyzer (6) connected to a circulation circuit (7) for the circulation of a dialyzer bath, an oxygenator (9) which is arranged on the circulation circuit (7), an acidifier (10), which is suited to introduce an acid substance into the circulation circuit (7) upstream of the oxygenator (9), and basic neutralization means (11, 12) which are arranged downstream of the dialyzer (6).

## Description

The present invention concerns an extracorporeal circuit for removing CO₂ from blood.

In the past, various solutions have been devised for removing CO₂ from blood. Many of these solutions entail extracorporeal treatment of the blood, analogously to the process of dialysis treatment which removes toxic or harmful substances which should normally be eliminated by the kidneys.

Removal of CO₂ from blood is necessary when the function of the lungs is damaged due to various pathologies and has to be substituted, even only partially.

In general, extracorporeal circuits for removing CO₂ from blood entail the venous blood being excluded from the lung circuit in order for it to be artificially oxygenated by means of a gas exchanger, such as an oxygenator. More specifically, the venous blood which returns towards the right atrium of the heart is collected in the extracorporeal circuit and pumped into the oxygenator from which it is then conveyed into the arterial circuit, thus by-passing the heart and the pulmonary circulation.

The most widely used oxygenation devices are membrane oxygenators in which the blood and the oxygen (or an oxygen-rich mixture) flow from opposite parts of a membrane. These types of devices therefore act on the quantity of CO₂ dissolved in the blood which, in reality, is a limited portion of the total CO₂ content. In fact, it is known that 90% of the CO₂ transported in the blood is in the form of carbonate ions according to the following chemical equilibrium (I).

CO₂ + H₂0 ↔ H₂CO₃ ↔ H⁺ + HCO₃⁻ (I)

One solution in order to increase the removal of CO₂ from blood by means of membrane oxygenators could be to intervene on the chemical equilibrium (I), inducing an increase in the percentage of gaseous CO₂. In this way, the increase in concentration of gaseous CO₂ to be removed by the oxygenator promotes removal of the total CO₂.

One way of intervening on the chemical equilibrium (I) in favour of the gaseous CO₂ is acidification from the outside. Said acidification is achieved by the addition of acid substance in the extracorporeal circuit upstream of the oxygenator.

As may seem obvious to a person skilled in the art, said addition of acid can entail a series of drawbacks which must be resolved in order not to endanger the health of the patient.

The object of the present invention is to produce an extracorporeal circuit for removing CO₂ from blood, able to carry out the above-mentioned acidification which promotes shifting of the equilibrium (I) in favour of the gaseous CO₂ and at the same time guarantees high safety levels.

The subject of the present invention is an extracorporeal circuit for removing CO₂ from blood, the essential characteristics of which are reported in claim 1, and the preferred and/or auxiliary characteristics of which are reported in claims 2-5.

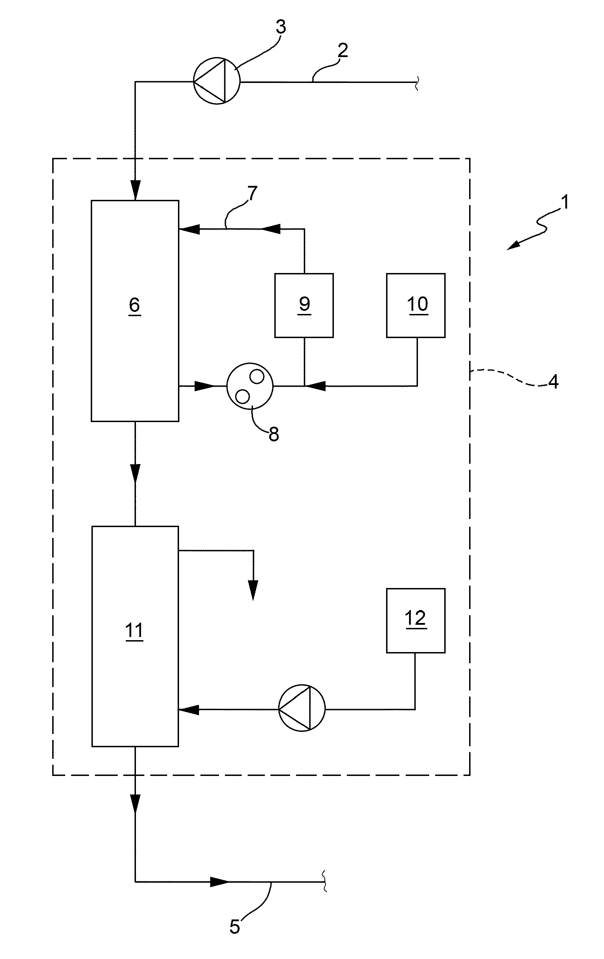

For a better understanding of the invention, an embodiment is provided below, for purely illustrative non-limiting purposes with the help of the accompanying figure, which schematically illustrates an extracorporeal circuit for removing blood according to the present invention.

In the figure, the number 1 indicates as a whole an extracorporeal circuit for removing blood subject of the present invention.

The circuit 1 comprises a blood taking line 2 for taking blood from the patient, on which a peristaltic pump 3, an oxygenation assembly 4 and a blood re-introduction line 5 for re-introducing the blood into the patient operate.

The oxygenation assembly 4 comprises a dialyzer 6 connected to a dialyzer bath circulation circuit 7 operated by a peristaltic pump 8 and an oxygenator 9 arranged on the dialyzer bath circulation circuit 7 and an acidifier 10 suited to introduce into the dialyzer bath circulation circuit 7 an acid substance upstream of the oxygenator 9 following the circulation flow. In particular, the dialyzer 6 is a haemofilter and the dialyzer bath is plasma water.

The acid substances introduced by the acidifier 10 cause a shift in the chemical equilibrium (I) in favour of the gaseous CO₂ in the plasma water in circulation. The gaseous CO₂ will then be removed by the action of the oxygenator 9.

In particular, the acid substance is a mixture of an inorganic acid, such as hydrochloric acid for example, and organic acids, such as pyruvic acid, citric acid and lactic acid already normally present in the organism.

The oxygenation assembly 4 furthermore comprises a dialyzer 11 arranged downstream of the dialyzer 6 from which it receives the blood. The dialyzer 11 entails the use of a basic dialyzing solution 12 such as to re-balance the pH modified by acidification of the plasma water in the dialyzer bath circulation circuit.

It has been proved that performing the acidification on the plasma water instead of directly on the blood guarantees a greater efficiency both in terms of effectiveness of the acidification itself and in terms of safety.

## Claims

1. An extracorporeal circuit (1) for removing CO₂ from blood comprising a blood taking line (2) for taking blood from the patient, an oxygenation assembly (4), and a blood re-introduction line (5) for re-introducing blood into the patient; said extracorporeal circuit being **characterised in that** said oxygenation assembly (4) comprises a first dialyzer (6) connected to a circulation circuit (7) for the circulation of a dialyzer bath, an oxygenator (9) which is arranged on the circulation circuit (7), an acidifier (10), which is suited to introduce an acid substance into the circulation circuit (7) upstream of the oxygenator (9), and basic neutralization means (11, 12) which are arranged downstream of the dialyzer (6).

2. The extracorporeal circuit (1) for removing CO₂ from blood according to claim 1, **characterised in that** the basic neutralization means comprise a second dialyzer (11), in which a respective basic dialyzing solution (12) circulates.

3. The extracorporeal circuit (1) for removing CO₂ from blood according to claim 2, **characterised in that** said circulation circuit (7) for circulation of the dialyzer bath is a circulation circuit for circulation of plasma water.

4. The extracorporeal circuit (1) for removing CO₂ from blood according to claim 3, **characterised in that** said first dialyzer (6) is a haemofilter.

5. The extracorporeal circuit (1) for removing CO₂ from blood according to any of the previous claims, **characterised in that** said oxygenator (9) is a membrane oxygenator.
